# EUROPEAN PATENT APPLICATION

(11) **EP 3 964 141 A1**
(43) Date of publication of application: **09.03.2022**
(21) Application number: 20194149.9
(22) Date of filing: 02.09.2020
(51) Int. Cl.: A61B 17/11

(54) **IMPLANT AND IMPLANT APPLICATOR FOR SURGICAL ANASTOMOSIS**

(71) Applicant: Technische Universität München, 80333 München (DE)
(72) Inventor: Steger, Jana, 80469 München (DE); Ostler, Daniel, 81371 München (DE); Wilhelm, Dirk, 80469 München (DE); Wohlgemuth, Sandra, 81371 München (DE); Ficht, Stefanie, 80798 München (DE); Eblenkamp, Markus, 85635 Höhenkirchen-Siegertsbrunn (DE); Mela, Petra, 85748 Garching bei München (DE)
(74) Representative: Lucke, Andreas

(57) **Abstract**

An implant for surgical anastomosis, the implant comprising a first implant component and a second implant component, wherein the first implant component and the second implant component comprise matching connection elements configured to hold the first implant component and the second implant component in a fixed relative spatial relationship and/or to compress body tissue arranged between the first implant component and the second implant component, wherein the first implant component and the second implant component form a through-going passage when the implant is deployed for surgical anastomosis, and wherein the first implant component and the second implant component are convertible between a first configuration and a second configuration, the first configuration and the second configuration defining different perimeters, to establish a perimeter of the through-going passage.

## Description

### FIELD OF THE INVENTION

The present invention is in the field of surgical support devices and implants. More precisely, the present invention relates to surgical devices for the transluminal creation of a surgical anastomosis.

### BACKGROUND

One of the most frequent surgical interventions in colorectal surgery is the resection of a pathological bowel section, such as the surgical removal of cancerous tissue. After the pathological bowel section has been removed, the resulting bowel endings need to be surgically reconnected to restore the natural function of the gastrointestinal tract. This connection of the two lumina is referred to as a surgical anastomosis.

Conventionally, the surgical anastomosis between the disconnected bowel sections is established by sutures or stapling techniques, thereby joining the bowel sections to each other at a plurality of joining points.

However, most available techniques to restore a fluid-tight connection between the tissue sections using sutures or staplers require open surgery and are associated with postoperative complications, such as stenosis or leakage, as well as surgical trauma. While the surgical trauma can be reduced through laparoscopic surgery techniques, the required surgical skill for these interventions can be significant. Recent efforts have therefore focused on supporting the surgical procedure by specialized devices, such as specialized staple applicators for transluminal insertion towards the anatomical site of the resection and for reducing the required surgical skill for the (surgical) intervention as well as the risk of surgical errors.

For example, US 6,503,259 B2 discloses a stapling system configured to pierce inverted tissue sections of both the oral and aboral lumen with a circular configuration of pins and receiving elements. The pins and receiving elements are arranged as a plurality of separate and unconnected staple elements in order to allow the anastomosis to remain flexible, while a pre-determined diameter of the surgical anastomosis is defined by a gasket held in place by the staple elements. A dispenser is provided which is inserted into the lumen and drives the pins into the receiving elements in a fixed spatial configuration in order to create a surgical anastomosis.

US 7,338,505 B2 discloses an end-to-end anastomosis stapling device comprising distal and proximal stapler portions, which are expandable to increase the accuracy of the anastomosis while allowing passage through the bowel.

As an alternative, surgical implants can be inserted into the anatomical cavities for compressing and joining aboral and oral tissue sections and for inducing and supporting the healing process.

For example, US 8,623,036 B2 discloses a device for creating an anastomosis using magnets (magnamosis device). The device comprises two ring-shaped implant components with permanent magnets mounted therein. The magnets are used to join and compress the disconnected bowel sections via magnetic forces when the tissue is draped over the respective implant components. The ring-shaped implant components feature mating surfaces having different curvatures in order to induce non-uniform compressive forces onto the tissue sections. Different dimensions of the anastomosis can be obtained by either providing differently shaped replicas of the implant components or using multiple implants in parallel.

US 10,517,600 B2 discloses a self-assembling magnetic anastomosis device formed by magnetic sections pivotally connected to each other. The pivotally connected sections enable storage and transport of the implant components in a folded or semi-linear state towards a target position. When the device is released, the internal spring forces of the joints bias the magnetic sections into a substantially circular shape of a self-assembling polygonal implant component. Two corresponding implant components may then be used to join disconnected tissue sections to create a surgical anastomosis via magnetic forces. Different shapes of the resulting implant can be pre-determined by the size and number of sections in the implant components.

### SUMMARY OF THE INVENTION

However, the known surgical implants generally provide only a set spatial configuration of the resulting implant supported anastomosis. Hence, a customized anastomosis adapted to the conditions of a patient, such as a certain colon diameter, requires the provision of a customized implant prior to the surgical procedure. At the same time, the approach of the implant towards the anatomical site of the intervention and the manipulation of the implant within the body have remained challenging.

On the other hand, creation of a surgical anastomosis using staples bears the risk of foreign material remaining in the body and inducing postoperative complications. Further, most of the prior art devices are not adapted for transluminal application, i.e. for the application via a natural orifice of the body to minimize surgical trauma, or said application is limited to proximal cavity sections due to the difficulty of force transmission along a winding path and through the lumen to deform metallic staples.

In view of this state-of-the-art, the object of the invention is to provide an improved system for end-to-end anastomosis compatible with transluminal surgery which does not rely on foreign material remaining permanently in the patient's body and which can provide a customized surgical anastomosis while reducing the required surgical skill for the intervention as well as the risk of surgical errors.

This object is solved by an implant for surgical anastomosis, an implant applicator and a kit of the implant and the implant applicator according to the independent claims. The dependent claims relate to preferred embodiments.

According to a first aspect, the invention relates to an implant for surgical anastomosis. The implant comprises a first implant component and a second implant component, wherein the first implant component and the second implant component comprise matching connection elements configured to hold the first implant component and the second implant component in a fixed relative spatial relationship and/or to compress body tissue arranged between the first implant component and the second implant component. The first implant component and the second implant component are configured to form a through-going passage when the implant is deployed for surgical anastomosis. The first implant component and the second implant component are further convertible between a first configuration and a second configuration, the first configuration and the second configuration defining different perimeters, to establish a perimeter of the through-going passage.

The surgical anastomosis can be created by inserting the first implant component into a first lumen to be joined to a second lumen via the surgical anastomosis, and by inserting the second implant component into the second lumen. Respective tissue walls of the first and second lumina may then be folded over the first implant component and the second implant component, respectively, to align the tissue walls on facing surfaces of the first implant component and the second implant component. The relative spatial positions of the implant components may then be fixed by connecting the first implant component and the second implant component via the matching connection elements to hold the tissue walls of the first and second lumina against each other. The through-going passage formed by the first implant component and the second implant component may enable the restoration of the natural biological function through the anastomosis with the implant holding the tissue walls against each other.

As opposed to prior devices, the implant can provide a selectable perimeter to facilitate the deployment of the implant and to enable the creation of a customized surgical anastomosis which can be adapted to the anatomical/physiological conditions of the patient. In particular, the first implant component and the second implant component may be converted from the first configuration to the second configuration during a surgical procedure, such as to facilitate the transluminal approach of the implant to the luminal margins and to simplify the following surgical procedure. For example, the first implant component and the second implant component may be transluminally approached to the anatomical site of resection in a first configuration featuring a small perimeter and may then be expanded to a second configuration featuring a larger perimeter than the first configuration.

As both the first implant component and the second implant component are convertible between the first configuration and the second configuration and may accordingly be adjusted to feature different perimeters, the manipulation of each implant component can be performed in a mirrored fashion to simplify the application of an implant transluminally and/or with customized dimensions. At the same time, the respective implant components can be independently controlled resulting in a versatile implant during application.

The perimeter may be adjusted by radially expanding each of the convertible implant components along a radial direction, such that the surrounding tissue wall of the first and second lumina may fold over axial facing surfaces of the implant components and may be aligned for the creation of the surgical anastomosis; the radial direction being perpendicular to the through-going passage defined by the implant components and the axial facing surfaces of the first implant component and the second implant component facing each other along the through-going passage.

Preferably, the perimeter should be adjustable by adjusting a spacing of adjacent connection elements of the first implant component and of the second implant component.

The first implant component and the second implant component should maintain their perimeters in the second configuration in the absence of external forces. By maintaining the established perimeter of the surgical anastomosis, a uniform healing process of the tissue sections may be stimulated while leakage may be prevented. Preferably, the first implant component and the second implant component are convertible for selecting and maintaining the inside perimeter of the first implant component and the second implant component, respectively, when the implant is deployed for surgical anastomosis in order to establish the perimeter of the through-going passage.

The skilled person will appreciate that a limited degree of flexibility can nonetheless be desirable to support the natural function of the tissue at the surgical anastomosis. Hence, the maintained perimeter in the absence of external forces should be understood to not exclude elastic deformation of the first implant component and the second implant component when the implant is deployed for surgical anastomosis. Rather, the maintained perimeter should be understood to constitute a target perimeter which can be selected by converting the first implant component and the second implant component between the first configuration and the second configuration, while the first implant component and/or the second implant component may feature a restoring force biasing the first implant component and/or the second implant component towards the maintained (target) perimeter. However, the first implant component and/or the second implant component may also be rigid to prevent deformation of the surgical anastomosis after converting the implant component(s) between the first configuration and the second configuration.

In addition, the skilled person will appreciate that the first implant component and the second implant component may be converted back and forth between different configurations featuring different perimeters during a (surgical) intervention, such as to determine a suitable implantation configuration. For example, the implant may be expanded for folding tissue walls over the implant components, and the implant components may subsequently be at least partially collapsed for connecting the matching connection elements. The implant components may subsequently be (re-)expanded to the suitable implantation configuration for establishing the through-going passage. The implant components may then maintain the perimeter of the through-going passage, when the implant is deployed for surgical anastomosis.

Additionally or alternatively, the perimeter and/or the shape of the implant components may be passively adjusted following deployment over a transitory period, such as by swelling or by deflating of the implant components due to an interaction with the surrounding environment. For example, absorbing components or biodegradable components of the implant components may progressively absorb body liquids or may be progressively degraded, respectively, such as to adapt to tissue swelling or deswelling at the anatomical site of the intervention. In some embodiments, the implant substantially maintains the perimeter of the through-going passage over a transitory period during a healing process of the surrounding tissue sections.

In some embodiments, absorbing portions of the implant components swell over a period of hours or days after the implant has been deployed for creating the surgical anastomosis, such that the implant is passively adjusted towards an anatomically possible perimeter and/or such that an axial pressure onto the body tissue is adjusted, e.g. to compensate for a subsiding postoperative tissue de-swelling.

Preferably, the first implant component and/or the second implant component are convertible to adjust a spacing of a plurality of connection elements along the perimeter of the respective implant component from the first configuration towards the second configuration and to lock the relative spatial configuration and/or the spacing of the connection elements in the second configuration, such that the locked relative spatial configuration and/or the locked spacing of the connection elements maintains the perimeter of the first implant component and/or the second implant component. Hence, the implant may be expanded at the anatomical site of the intervention while the connection elements maintain a relative alignment along the perimeter of the respective implant component for creating the surgical anastomosis.

In preferred embodiments, the first implant component and the second implant component are configured to maintain a perimeter selected from a range of supported perimeters when the implant is deployed for anastomosis.

For example, the first implant component and the second implant component may be continuously adjustable between different supported configurations which cover a range of supported perimeters to adapt the implant to the conditions of the patient. In some embodiments, the first implant component and the second implant component can be stored in a transport configuration to be transluminally approached to the anatomical site of the intervention and can then be expanded to a deployed configuration, wherein a perimeter of the implant component in the deployed configuration can be selected from the range of supported perimeters and wherein the first implant component and the second implant component maintain the selected perimeter in the absence of external forces. For example, the perimeter of the first implant component and the second implant component may be expanded, such that a diameter of the through-going passage is increased by at least 10%, preferably by at least 20%, such as by 30%.

In preferred embodiments, the first implant component and the second implant component are configured to transfer and/or to distribute compressive forces on body tissue held between the first implant component and the second implant component, such that a healing process of the body tissue is stimulated along a closed loop around the perimeter of the through-going passage when the implant is deployed for supporting a surgical anastomosis. Hence, the implant may induce and support a surgical anastomosis and may prevent leakage from the site of the anatomical intervention. The body tissue held between the first implant component and the second implant component may grow together to form the surgical anastomosis. The implant may be removed from the surgical anastomosis when the body tissue sections of the first and second lumina adhere to each other. In some embodiments, the implant components may be configured to collapse after a predetermined period of time and may be excreted. For example, the implant components may feature a predetermined breaking point formed of a biodegradable material and/or may be filled with a biocompatible material which may be (progressively) leaked, degraded or absorbed, such that the implant components separate from the surrounding body tissue after a predetermined period of time when the structural integrity of the implant components is sufficiently reduced.

When the implant is deployed for surgical anastomosis, the holding force holding the first implant component against the second implant component by the matching connection elements may also overcome a threshold for inducing necrosis in at least portions of the clamped tissue sections arranged between (the axial surfaces of) the first implant component and the second implant component. Tissue sections joined by the implant components may then adhere to each other and grow together for forming the surgical anastomosis while clamped tissue sections compressed between (the axial surfaces of) the implant components may necrotize and subsequently separate from the surrounding tissue. The implant may then separate from the surrounding tissue by release of the necrotic tissue and may be excreted with the separating necrotized tissue clamped by the implant components.

In some embodiments, the first implant component and the second implant component are configured to transfer and/or to distribute compressive forces on body tissue held between the first implant component and the second implant component, such that necrosis of the body tissue is effected along a closed loop around the perimeter of the through-going passage when the implant is deployed for surgical anastomosis.

The first implant component and the second implant component may feature a plurality of connection elements distributed along the perimeter of the first implant component and the second implant component such that compressive forces are distributed around the perimeter of the through-going passage to promote uniform necrosis of the tissue at the surgical anastomosis for independent separation of the implant from the surrounding tissue as well as uniform healing of the joined tissue sections surrounding the necrotizing tissue.

In preferred embodiments, the first implant component and the second implant component comprise an implant body circumscribing the through-going passage in a closed loop, wherein the implant body defines the perimeter in the first configuration and in the second configuration.

The continuous implant body circumscribing the through-going passage in a closed loop may apply uniform pressure from both sides to clamped tissue portions. The uniform pressure may limit leakage from the surgical anastomosis and may also induce a uniform healing process along the closed loop. In addition, necrosis may be induced through the clamping pressure in the closed loop, such that the implant may reliably separate from the surgical anastomosis.

In preferred embodiments, the matching connection elements are fixedly attached to the implant body.

With the matching connection elements fixedly attached to the implant body, the number of manipulated components of the implant can be limited and the application of the implant can be facilitated.

In preferred embodiments, the first implant component and the second implant component comprise a plurality of rigid sections interconnected by adjustable joining sections, wherein the first implant component and the second implant component are convertible between the first configuration and the second configuration by adjusting the joining sections to establish a different spacing between the plurality of rigid sections in the first configuration and in the second configuration.

The rigid sections and the joining sections may mutually form the implant body circumscribing the through-going passage in a closed loop. The different spacing of the rigid sections may adjust the perimeter of the first implant component and the second implant component for converting the first implant component and the second implant component between the first configuration and the second configuration.

The rigid sections may comprise the connection elements. Hence, an applicator may mount the implant via the rigid sections associated with the connection elements to define a relative connection geometry, while adjustment of the joining sections may enable an operator to select a perimeter of the first implant component and the second implant component to establish a perimeter of the though-going passage when the implant is deployed for anastomosis.

In preferred embodiments, the joining sections are configured to maintain the spacing between the plurality of rigid sections when the perimeter of the through-going passage is established.

In other words, the joining sections may maintain a spacing between the plurality of rigid sections when the implant is deployed for surgical anastomosis.

In preferred embodiments, the joining sections comprise a shape setting element, in particular a convertible cavity for receiving a filling medium and/or a locking mechanism and/or a compressible elastic element, to establish the perimeter of the through-going passage.

The shape setting element may adjust and maintain a spacing of the rigid sections to set a perimeter of the first implant component and the second implant component.

The convertible cavity may be filled with the filling medium when the implant is deployed for surgical anastomosis, such that the convertible cavity is expanded by the filling medium to adjust the spacing between the rigid sections. For example, the convertible cavity may comprise a flexible conduit connecting the rigid sections and may be expandable to adjust the spacing between the rigid sections. The joining sections may be coupled to a manipulation port of the respective implant component configured to receive the filling medium and to guide the filling medium to the convertible cavity. The filling medium filling the convertible cavity may maintain the spacing of the rigid sections by preventing a collapse of the convertible cavity.

The filling medium may comprise polymerizing solutions or gels for maintaining the spacing of the rigid sections with crosslinked polymers, such as gels or solutions of superabsorbent polymers, e.g. a Hydrogel, orthophosphates and/or epoxides, which can be injected into the convertible cavity for expanding the joining sections and for maintaining a continuously adjustable spacing between the rigid sections. The implant components may comprise a liquid port to connect to a fluid conduit for receiving the filling medium. The liquid port may be associated with a valve element for preventing a discharge of the filling medium from the convertible cavity. In some embodiments, the filling medium hardens for maintaining the spacing between the rigid sections after expansion of the convertible cavity and for preventing a discharge of the filling medium from the convertible cavity. In addition, absorbing or hardening additives may be provided in cavities of the implant components to absorb body liquids and to passively adjust a shape of the implant or compressive force on the tissue sections after implantation.

The filling medium may harden via a polymerization process, such as the hardening process of bone cement, or may harden by fluid absorption and a corresponding viscosity increase, as in the case of a hydrogel. Hence, in the context of the implant components the term "hardening" should be understood broadly to refer to a change of the elastic properties of the filling medium after the filling medium has been filled into the convertible cavity, such that the implant components may establish the perimeter of the through-going passage, not limited to a polymerization process or phase transition.

The implant components may comprise a convertible cavity forming a closed loop around the through-going passage, and the rigid sections may comprise internal fluid connections to connect different sections of the convertible cavity. In some embodiments, the implant components comprise a plurality of convertible cavities arranged between the rigid sections wherein the convertible cavities each are connectable to fluid conduits for individually receiving the filling medium.

In some embodiments, the rigid sections may be reinforced sections of an elastic implant body comprising a convertible cavity, wherein the matching connection elements may be attached to the reinforced sections.

For example, the implant components may comprise an elastic and at least partially hollow implant body, such as an elastic tube which may be reinforced by elastic biasing elements, e.g. an elastic spiral element, and the elastic body may feature reinforced sections associated with the matching connection elements. The implant components may then be deployed by expanding the implant components via a restoring force of the elastic body from a storage/transport configuration towards an expanded configuration featuring a larger perimeter than the storage/transport configuration. The implant components may be adjusted via further expansion of the convertible cavity, e.g. by filling the convertible cavity with the filling medium.

The elastic biasing elements may be configured to provide different elastic moduli along different spatial dimensions when deformed by the filling medium, such that the force imparted by the filling medium asymmetrically adjusts the thickness and the perimeter of the implant components. For example, the thickness of the implant components may remain substantially constant while the perimeter of the implant components is adjusted via the filling medium, or vice-versa, or a thickness of the implant components may be adjusted substantially along the axial direction of the implant components, i.e. along the through-going passage, and/or the radial direction when the implant components are filled with the filling medium. Additionally or alternatively, the filling medium or absorbing/hardening additives arranged in the implant components may swell to expand the implant components following implantation, such as to progressively expand the implant components following deployment and/or to passively adapt the implant to the anatomical conditions. For example, the implant components may be configured to passively adjust the shape towards an anatomically possible perimeter and/or to compensate for a subsiding postoperative tissue swelling along the radial direction and/or the axial direction.

Alternatively or additionally, the joining sections may comprise a locking mechanism convertible between different configurations to mechanically adjust and lock a spacing between the rigid sections. For example, the locking mechanism may comprise two interlocking sections respectively connected to adjacent rigid sections, such as a locking pin attached to a first rigid section and a rack featuring form-locking recesses attached to a second rigid section, wherein the pin may selectively interlock with the form-locking recesses to adjust and lock a spacing between the rigid sections. In some embodiments, the locking mechanism is configured to allow relative movement between the rigid sections in an expansion direction and to prevent relative movement in the opposite direction, e.g. by comprising asymmetrically formed interlocking sections, such that the implant component may be expanded during deployment but prevents a subsequent collapse of the through-going passage after the target perimeter has been established.

Alternatively or additionally, the implant components may comprise a compressible elastic element to bias the implant component towards a target perimeter when the implant has been deployed for surgical anastomosis. For example, the joining sections may comprise spring elements for insertion into a lumen, which are released to irreversibly expand the implant component towards a target shape/perimeter. In some embodiments, the spring elements comprise a shape-memory alloy which is cooled below a transformation temperature and compressed for approaching the anatomical site of the intervention. Upon heating, the shape-memory alloy may cross the transformation temperature to bias the spring element towards a larger perimeter. Hence, the implant components may be expanded without an external mechanical force (e.g. cable pull transmitted forces, pneumatic forces or hydraulic forces) or magnetic force by the biasing force of the compressible elastic element. In some embodiments, the implant body is elastic and is biased to assume an expanded shape, e.g. the implant body may comprise an elastic hollow tube section featuring a spiral spring integrated with the elastic hollow tube section, for biasing the implant body towards an expanded diameter.

The shape setting elements may maintain the spacing of the rigid sections when the shape is converted to a target shape for deployment in order to establish the perimeter of the through-going passage and to maintain the shape of the implant when deployed for anastomosis.

The perimeter of the implant components may be defined by the length of the rigid sections and the joining sections including the shape setting elements, such as conduits or mechanical adjustment systems, or may be defined by the effective inside perimeter of the implant components in the first configuration and the second configuration, wherein the effective inside perimeter effectively establishes the perimeter of the through-going passage.

In preferred embodiments, the rigid sections are formed by or mount the matching connection elements of the first implant component and the second implant component.

The connection elements may be formed of force locking or form locking connection elements, and may include magnetic portions (e.g. permanent magnets) or magnetizable portions (e.g. ferromagnetic material), form locking pins or recesses, clamp brackets, brackets or openings for receiving sutures, or any combination thereof for holding the first implant component and the second implant component in a fixed relative spatial relationship and/or for compressing body tissue at the surgical anastomosis. For example, the implant components may feature openings for piercing the clamped tissue sections with barbed sutures for relative fixation of the tissue sections and/or for holding the implant components and the tissue sections in a fixed relative spatial relationship. In some embodiments, the connection elements are of a non-piercing type to connect matching connection elements of the first implant component and the second implant component without requiring piercing of the joined tissue sections, such that a force transmission of a tissue piercing force to the site of the surgical intervention can be avoided. For example, a clamp may engage clamp brackets of the implant components from a radially inwards side of the implant components for holding the implant components in a fixed relative spatial relationship.

In preferred embodiments, the matching connection elements of at least one of the first implant component and the second implant component comprise a permanent magnet to magnetically attract a magnetic or magnetizable portion, in particular an inversely polarized permanent magnet, of the respective other matching connection element.

Magnetic connection elements enable the alignment and locking of the implant components to each other while compressing tissue sections lying in between the implant components without the application of external mechanical forces and without piercing the compressed tissue sections. Hence, the implant may be deployed also in deep cavities where controlled connection of the implant components via mechanical forces transmitted through the lumen would be challenging. In addition, magnetic connection elements may self-align and may thus facilitate application of the implant.

Alternatively or additionally, the connection elements may feature mechanical connection elements such as clamps for clamping the first implant component and the second implant component via the through going passage, or by driving a mechanical locking element past or through the tissue sections to mechanically link the connection elements.

In preferred embodiments, the first implant component and the second implant component further comprise mounting elements to mount the first implant component and the second implant component to an implant applicator on the inner perimeter of the first implant component and the second implant component, respectively.

The mounting elements may be arranged on the inner perimeter of the first implant component and/or the second implant component or may be arranged on the side surfaces adjacent to the inner perimeter of the first implant component and/or the second implant component, in particular on a side opposite of the matching connection elements, such as to mate with a matching mounting support on an outer face of an implant applicator. The mounting elements may comprise magnetic or magnetizable elements, such as a permanent magnet or a ferromagnetic material section, to magnetically mount the first implant component and/or the second implant component on a corresponding magnetic or magnetizable element of the implant applicator. Additionally or alternatively, the mounting elements may feature clamping brackets to engage with a clamping element of the implant applicator, such as to mechanically mount the first implant component and/or the second implant component on the implant applicator.

The mounting elements may hold the first implant component and the second implant component on the outer face of the implant applicator during an approach of the implant towards the anatomical site of the intervention and during an alignment of the respective tissue walls over the axial facing surfaces of the implant components. When the first implant component is connected to the second implant component via the matching connection elements, the implant applicator may be disconnected from the implant components and may be retracted through the through-going passage formed by the implant. In some embodiments, a radial displacement of the mounting supports of the implant applicator can be used to expand the perimeter of the through-going passage and to convert the first implant component and the second implant component between the first configuration and the second configuration. Hence, the implant may be simple to use and to deploy.

In addition, manipulation ports of the implant components, such as fluid ports for receiving a filling medium or mechanical actuators, to convert the first implant component and the second implant component between the first configuration and the second configuration may be integrated with and/or arranged in a fixed spatial relationship with the mounting elements, such as to control the perimeter of the first configuration and the second configuration via applicator elements attached to the mounting support. The perimeter of the implant may then be reliably changed through the applicator elements while the first implant component and the second implant component is mounted to the implant applicator via the mounting elements, even if the position of the mounting supports is changed in accordance with the change of the configuration of the implant components.

In a second aspect, the invention relates to a kit comprising the implant of the first aspect and an implant applicator comprising relatively displaceable mounting supports to releasably mount the first implant component and the second implant component in a spatially separated arrangement, and to approach the first implant component and the second implant component to each other for surgical anastomosis.

In a third aspect, the invention relates to an implant applicator for clamping surrounding tissue sections with a pair of implant components to perform surgical anastomosis. The implant applicator comprises an elongated applicator body, a pair of implant manipulators, an implant expanding mechanism, and a drive mechanism. The pair of implant manipulators is configured to be displaced with respect to each other along the applicator body, and the drive mechanism is configured to move one or both of the implant manipulators of the pair of implant manipulators towards each other. Each implant manipulator of the pair of implant manipulators comprises a mounting support to selectively hold a respective implant component of the pair of implant components. The implant expanding mechanism is configured for selectively increasing an inner perimeter of one or both implant components of the pair of implant components mounted on the mounting support.

The elongated applicator body should be configured for transluminal approach towards the tissue sections and may accordingly feature flexible or flexibly connected segments to navigate through an anatomical lumen, which may be selectively stiffened. In preferred embodiments, the elongated applicator body comprises an accommodating passage to mount the implant applicator on the outer face of an endoscope. Hence, the implant applicator may be combined with the endoscope to navigate through an anatomical lumen. Additionally or alternatively, the implant applicator may comprise an imaging device configured to transmit an image from the distal portion of the implant applicator to a proximal portion of the implant applicator, such as to integrate the functionality of an endoscope with the applicator or to provide additional imaging capabilities, e.g. for supporting an operator while creating the surgical anastomosis.

In preferred embodiments, the outer diameter of the implant applicator is smaller than 60 mm, in particular smaller than 40 mm, preferably smaller than 30 mm in a navigation configuration of the implant applicator to navigate through an anatomical lumen. In the navigation configuration, the implant manipulator may be held against the elongated applicator body to minimize the outer circumference of the implant applicator while the anatomical lumen, e.g. a human colon, is navigated. Preferably, the outer diameter of the implant applicator is smaller than 40 mm, most preferably smaller than 30 mm, in the navigation configuration including the space occupied by the implant components mounted on the mounting supports.

The mounting support may be arranged on a radially outward face of the implant manipulator to mount the implant components on an outer perimeter of the implant applicator. When the implant applicator approaches the anatomical site of the intervention with the implant manipulators, disconnected proximal and distal tissue sections, e.g. oral and aboral sections of the bowel, may be folded over the implant components on the outer perimeter of the implant applicator to align the respective proximal and distal tissue sections with respect to each other. The drive mechanism may then be driven to move the implant manipulators towards each other, thereby clamping the folded proximal and distal tissue sections between the implant components mounted on the implant manipulators. The tissue sections may be folded over the implant components and/or the implant manipulators with laparoscopic assistance and may be held against the implant components via sutures, clamps or hooks on the implant components or the implant manipulators.

In preferred embodiments, each implant manipulator of the pair of implant manipulators further comprises a tissue folding section for at least partially folding tissue of a lumen surrounding the implant manipulator, such that the tissue of the lumen is aligned for engagement with the implant component held by the implant manipulator along the linear displacement axis of the elongated applicator body.

The pair of implant manipulators may be driven to expand radially away from the elongated applicator body, such that the tissue sections are folded along the tissue folding section, such as hooks or contoured sections of the implant manipulators.

In some embodiments, the pair of implant manipulators comprises pivotally hinged manipulator arms to pivot with respect to the elongated applicator body such that the tissue folding section is radially displaced away from the elongated applicator body to fold the tissue of the surrounding lumen.

The pivotal motion of the manipulator arms may be driven with any suitable mechanical drive mechanism, such as a cable pull connected to the implant applicator or may be driven with hydraulic forces applied through fluid conduits from a proximal side of the implant applicator, to actuate the pivot mounting of the pair of implant manipulators on the implant applicator. For example, a worm gear mounting of the implant manipulator may be driven by rotating or displacing a worm shaft on the elongated applicator body to drive a pivoting motion of the manipulator arms coupled to the worm shaft by a worm wheel. As another example, a displaceable runner on the elongated applicator body may be driven to pivot the pivotally mounted implant manipulators via stretchers similar to the opening mechanism of an umbrella.

In preferred embodiments, the implant expanding mechanism is configured to radially displace the mounting support of one or both implant manipulators away from the elongated applicator body, wherein the pair of implant manipulators in particular comprises pivotally hinged manipulator arms to pivot with respect to the elongated applicator body such that the mounting support is radially displaced away from the elongated applicator body.

The motion of the manipulator arms may radially displace the implant components mounted on the implant manipulators, such that the tissue sections are folded over the implant components via an elastic restoring force of the tissue. Preferably, the tissue sections are held in place on the implant manipulators while the drive mechanism approaches the pair of implant manipulators towards each other.

In some embodiments, a first implant manipulator of the pair of implant manipulators is actively driven to radially displace the implant components mounted on the associated implant manipulator, and the other implant manipulator of the pair of implant manipulators may passively follow a radial movement of the first implant manipulator, e.g. due to a force transfer via connected implant components.

Additionally or alternatively, the implant expanding mechanism may be driven to increase an inner perimeter of one implant component of the pair of implant components mounted on the mounting support, and the other implant component may be passively converted through a force transfer via matching connection elements holding the implant components in a fixed relative spatial arrangement.

In preferred embodiments, each implant manipulator of the pair of implant manipulators comprises a suction port at the tissue folding section, wherein the implant applicator comprises a fluid conduit for applying a suction force to the suction port.

The suction force may fold and/or reliably hold the tissue folded over the implant component and may be externally applied on a proximal end of the implant applicator via the fluid conduit. Preferably, the suction port is arranged radially inwards with respect to the mounting support and/or the implant component, such that holding the tissue sections at the suction port of the implant manipulator aligns the tissue sections over the implant component for creation of the surgical anastomosis.

In preferred embodiments, the implant expanding mechanism comprises fluid conduits to transfer a fluid to a cavity of each implant component of the pair of implant components, and/or an actuation assembly coupled to the pair of implant manipulators and configured to change a radial distance of the respective mounting support to the elongated applicator body by mechanical displacement of the pair of implant manipulators.

For example, the implant components may be mounted on an outer face of the implant manipulators and a pivoting motion of the implant manipulators may serve as the implant expanding mechanism by radially expanding the implant components mounted on the implant manipulators. The radial motion may induce a change of the inner perimeter of the implant component while a self-locking mechanism of the implant component may maintain the inside perimeter when the implant is deployed for surgical anastomosis. Additionally or alternatively, a filling medium may be transferred to the cavity of each implant component to inflate the implant components. In some embodiments, the implant applicator further features an actuator to drive a locking mechanism of the implant components, such as by driving a motion of a locking pin to lock a spatial configuration of the implant component. When the implant components have been expanded, the implant expanding mechanism may be disconnected from the implant components, such as by disconnecting the fluid conduit of the implant expanding mechanism from a corresponding fluid port of the implant component.

In some embodiments, the implant applicator further comprises an irradiation source, such as a UV-lighting source, to drive a hardening process of a filling medium in convertible fluid cavities of the implant components.

In preferred embodiments, the implant applicator further comprises a release actuator to release a mounting of the implant components to the mounting supports of the implant manipulators.

For example, the release actuator may comprise a force transmitter to apply a mechanical force generated at a proximal end of the implant applicator at the implant manipulators, such that a mounting can be selectively released by releasing or overcoming a force-locking and/or form-locking mounting of the implant components, e.g. by forcefully retracting the implant manipulators towards a radially inward configuration after the implant expanding mechanism has expanded the implant.

In preferred embodiments, the mounting supports comprise magnetic or magnetizable portions to mount the respective implant components via attracting magnetic forces, in particular a permanent magnet or an electromagnet.

In preferred embodiments, the release actuator is configured to mechanically retract or pivot the magnetic or magnetizable portions to reduce a magnetic force exerted by the mounting supports onto the implant components and/or to electrically invert or reduce a magnetic force exerted by the mounting supports onto the implant components.

For example, an electromagnet of the implant applicator may be driven to reduce an attractive force onto the implant components and/or to repel the implant components. As another example, a pin may be mechanically driven to protrude from the mounting support in order to space the implant manipulator and the implant component and to release the mounting.

In preferred embodiments, the drive mechanism comprises a fluid conduit for applying a hydraulic or pneumatic force from the proximal portion of the implant applicator to a distal portion of the implant applicator; and/or a cable pull (e.g. a Bowden cable) to transmit a mechanical force from the proximal portion of the implant applicator to a distal portion of the implant applicator; and/or an elastic element mounted on the elongated applicator body and biasing at least one implant manipulator of the pair of implant manipulators.

The skilled person will appreciate that the reference to a "pair" of implant manipulators and the corresponding "pair" of implant components is made for the sake of conciseness and does not imply any symmetry or similarity between the implant manipulators or implant components, respectively. Instead, reference is made to a pair of implant manipulators and of implant components solely to group cooperating elements which provide complementary functionality for creating the surgical anastomosis.

In a fourth aspect, the invention relates to a kit of an implant according to the first aspect and an implant applicator according to the third aspect, wherein the implant applicator of the third aspect is configured for surgical anastomosis using the implant of the first aspect.

In a fifth aspect, the invention relates to the use of the implant according to the first aspect and/or the implant applicator according to the third aspect in an intervention for creating a surgical anastomosis.

In a sixth aspect, the invention relates to a method for creating a surgical anastomosis with a convertible implant, the method comprising mounting a pair of implant components of the convertible implant on outer faces of a corresponding pair of implant manipulators of an implant applicator, approaching the pair of implant manipulators with the pair of implant components transluminally to an anatomical site associated with the surgical anastomosis, actuating an implant expanding mechanism and a drive mechanism of the implant applicator for expanding a perimeter of both implant components and for approaching the implant manipulators towards each other to create the surgical anastomosis, and retracting the implant applicator through a through-going passage formed by the implant components.

The skilled person will appreciate that the drive mechanism and the implant expanding mechanism may be actuated in any temporal order. For example, the implant components may be expanded prior to approaching the implant components and after creating the surgical anastomosis, such as to adjust a perimeter of the through going passage after the surgical anastomosis has been established. The implant may be any implant according to the first aspect, and the implant applicator may pertain to any implant applicator of the third aspect.

### DETAILED DESCRIPTION OF EMBODIMENTS

The features and numerous advantages of the implant and corresponding applicator according to the present invention will best be understood from a detailed description of preferred embodiments with reference to the accompanying drawings, in which:
- Fig. 1A, 1B: schematically illustrate an example of a convertible implant for surgical anastomosis;
- Fig. 2A-2C: schematically illustrate an example of a convertible implant and corresponding implant applicator for surgical anastomosis;
- Fig. 3A, 3B: schematically illustrate another example of a convertible implant for surgical anastomosis comprising convertible fluid cavities;

- Fig. 4A, 4B: schematically illustrate a further example of a convertible implant for surgical anastomosis comprising mechanically linked segments;
- Fig. 5A, 5B: schematically illustrate an example of a mechanical locking mechanism for a convertible implant;
- Fig. 6A: schematically illustrate a further example of a convertible implant for surgical anastomosis;
- Fig. 6B: schematically illustrate another example of a convertible implant for surgical anastomosis;
- Fig. 7A, 7B: schematically illustrate an example of an implant applicator for a convertible implant;
- Fig. 8A-8D: schematically illustrate a further example of an implant applicator for a convertible implant; and
- Fig. 9A-9D: schematically illustrate another example of an implant applicator for a convertible implant.

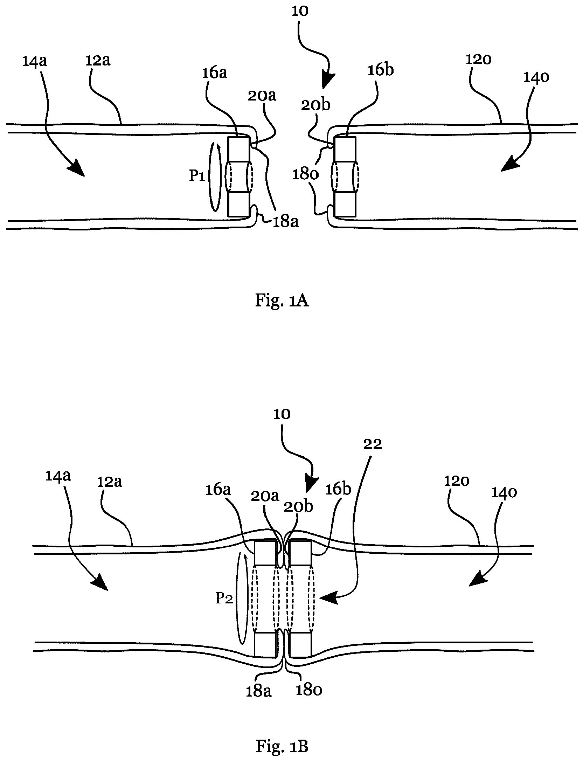

Fig. 1A and 1B schematically illustrate an example of a convertible implant 10 for surgical anastomosis depicted in the context of surgically disconnected bowel sections 12a, 120. The disconnected bowel sections 12a, 120 consist of an aboral tissue section 12a and an oral tissue section 120 forming an aboral lumen 14a and an oral lumen 140, respectively. The implant 10 comprises a first implant component 16a, and a second implant component 16b. The first implant component 16a is located in the aboral lumen 14a and the second implant component 16b is located within the oral lumen 140.

In Fig. 1A folded tissue sections 18a, 180 of the aboral tissue section 12a and the oral tissue section 120 are folded over facing surfaces 20a, 20b of the first implant component 16a and the second implant component 16b, respectively. The facing surfaces 20a, 20b are aligned with each other, such that the folded tissue sections 18a, 180 of the aboral tissue section 12a and the oral tissue section 120 are substantially aligned in parallel and face each other. The implant components 16a, 16b are in a first configuration, wherein the implant components 16a, 16b each feature a first perimeter Pi, which may be smaller than the perimeter of the aboral lumen 14a and the oral lumen 140.

In Fig. 1B, the first implant component 16a and the second implant component 16b have been expanded towards a larger second perimeter P2, larger than the perimeter of the aboral lumen 14a and the oral lumen 140, by converting the first implant component 16a and the second implant component 16b from the first configuration towards a second configuration. The first implant component 16a and the second implant component 16b may stretch the surrounding tissue sections 12a, 120, such as to assist a folding of the folded tissue sections 18a, 180 and/or to hold the folded tissue sections 18a, 180 aligned over the first implant component 16a and the second implant component 16b.

In addition, the first implant component 16a and the second implant component 16b are moved towards each other, such that the folded tissue sections 18a, 180 of the aboral tissue section 12a and the oral tissue section 120 are clamped to each other by the facing surfaces 20a, 20b of the first implant component 16a and the second implant component 16b located on opposite sides of the respective folded tissue sections 18a, 180. Matching connection elements (not shown in Fig. 1A, 1B) can be employed to hold the first implant component 16a and the second implant component 16b in a fixed relative spatial relationship while the surgical anastomosis may be formed by a healing process of joined tissue sections 12a, 120 surrounding the implant 10.

Each of the implant components 16a, 16b features an implant body circumscribing a thorough-going passage 22 in a closed loop. When the implant 10 is deployed for surgical anastomosis as schematically illustrated in Fig. 1B, the first implant component 16a and the second implant component 16b mutually form a through-going passage 22 connecting the aboral lumen 14a and the oral lumen 140 in order to restore the natural function of the bowel.

The implant 10 may in principle be installed via conventional surgical methods, such as open surgery or laparoscopic surgery. However, the implant 10 is preferably configured for transluminal application to be approached towards the resected bowel section via the aboral lumen 14a or the oral lumen 140.

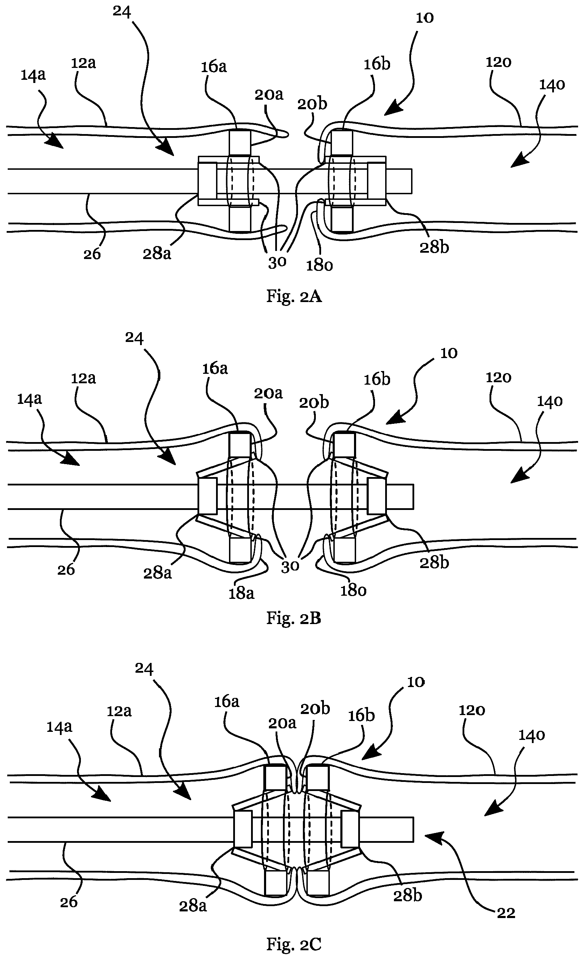

Fig. 2A-2C schematically illustrates an example of an implant applicator 24 for transluminally assisted surgery and a corresponding convertible implant 10 in different stages of the creation of a surgical anastomosis and in the context of surgically disconnected bowel sections 12a, 120 defining an aboral lumen 14a and an oral lumen 140. The implant applicator 24 comprises an elongated applicator body 26 as well as a first implant manipulator 28a and a second implant manipulator 28b arranged at different positions along the elongated applicator body 26 and facing each other. The first implant manipulator 28a mounts a first implant component 16a of the convertible implant 10 and the second implant manipulator 28b mounts a second implant component 16b of the convertible implant 10 via mounting supports on respective manipulator arms 30.

In Fig. 2A, the implant applicator 24 has been transluminally inserted via the aboral lumen 14a and protrudes into the disconnected oral lumen 140 with a distal portion of the implant applicator 24, the distal portion comprising the second implant manipulator 28b. The first implant manipulator 28a is arranged proximally with respect to the second implant manipulator 28b and is located within the aboral lumen 14a. The first implant component 16a and the second implant component 16b are located within the aboral lumen 14a and the oral lumen 140, respectively, in accordance with the positioning of the implant manipulators 28a, 28b. A folded section 180 of the oral tissue section 120 is folded over the second implant component 16b and held in the folded position at the manipulator arms 30 of the second implant manipulator 28b. Both the first implant component 16a and the second implant component 16b are in a first configuration featuring a first perimeter Pi.

In Fig. 2B, folded sections 18a, 180 the aboral tissue section 12a and the oral tissue section 120 are folded over the first implant component 16a and the second implant component 16b, respectively, and are held by the respective first and second implant manipulators 28a, 28b, such that the folded tissue sections 18a, 180 are aligned over facing surfaces 20a, 20b of the first and second implant component 16a, 16b.

In addition, the manipulator arms 30 of the first implant manipulator 28a and the second implant manipulator 28b are pivoted away from the elongated applicator body 26, such that the mounting supports mounting the first implant component 16a and the second implant component 16b are radially displaced away from the elongated applicator body 26. In accordance with the configuration of the implant manipulators 28a, 28b, the first implant component 16a and the second implant component 16b are converted towards a second configuration featuring a larger second perimeter P2.

In Fig. 2C, the first implant manipulator 28a and the second implant manipulator 28b are moved towards each other, such that the first implant component 16a and the second implant component 16b are approached towards each other and clamp folded tissue sections 18a, 180 aligned over the facing surfaces 20a, 20b of the first and second implant components 16a, 16b.

Matching connection elements (not shown in Figs. 2A-2C) can be employed to hold the first implant component 16a and the second implant component 16b in a fixed relative spatial relationship. When the first implant component 16a and the second implant component 16b are connected to each other, a mounting of the implant components 16a, 16b on the implant manipulators 28a, 28b via the manipulator arm 30 may be disconnected, such that the implant applicator 24 may be removed from the bowel. For example, the manipulator arms 30 may be pivoted towards the elongated applicator body 26 and the implant applicator 24 may be retracted through the through-going passage 22 formed by the implant components 16a, 16b and the aboral lumen 14a.

Hence, the implant 10 may be applied and manipulated transluminally through a natural lumen 14a of the body to assist a surgeon in the creation of a surgical anastomosis with minimal surgical trauma, while the implant components 16a, 16b are aligned with each other for mutual engagement during the approach and the expansion of the implant components 16a, 16b.

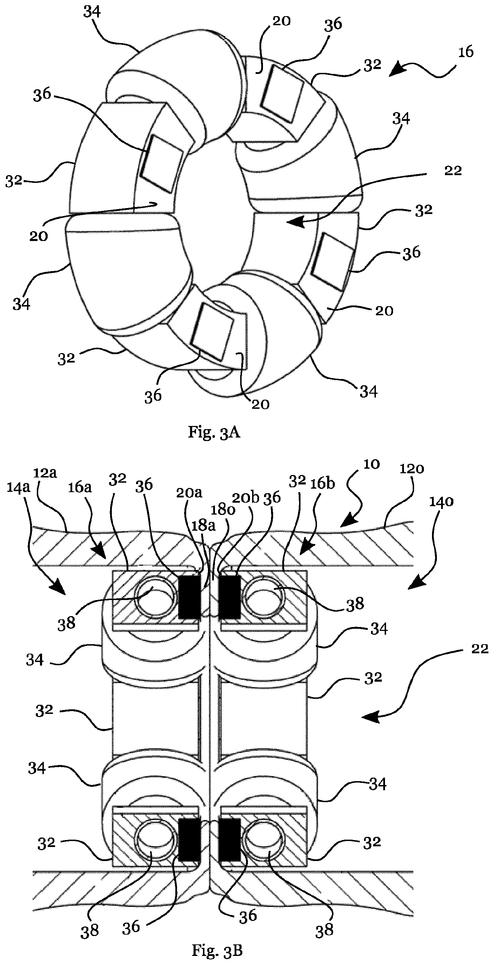

Fig. 3A, 3B show an example of a convertible implant 10 illustrated by a schematic perspective view of a convertible implant component 16 and a schematic illustration of the implant 10 in an application for surgical anastomosis.

Fig. 3A illustrates the implant component 16 convertible between a first configuration and a second configuration to adjust a perimeter Pi, P2 of the implant component 16. The implant component 16 comprises four rigid sections 32 joined to each other by joining sections 34 to form an implant body circumscribing a through-going passage 22 in a closed loop. The joining sections 34 comprise convertible cavities configured to receive a filling medium in order to expand the joining sections 34 and to adjust a spacing between the rigid sections 32. Connection elements are arranged on the axial faces 20 of the rigid sections 32 which are oriented along the through-going passage 22 defined by the implant component 16. Specifically, the implant component 16 comprises permanent connection magnets 36 mounted on the rigid sections 32 of the implant component and aligned along the through-going passage 22 to provide a connection element for clamping tissue sections 18a, 180 between matching implant components 16, 16a, 16b and for holding the implant components 16, 16a, 16b in a fixed relative spatial relationship.

Fig. 3B illustrates a sectional view of a first implant component 16a and a second implant component 16b in the context of surgically disconnected bowel sections 12a, 120 defining an aboral lumen 14a and an oral lumen 140. The first implant component 16a is located within the aboral lumen 14a and the second implant component 16b is located within the oral lumen 140 while folded tissue sections 18a, 180 of the oral and aboral tissue sections 12a, 120 are folded over facing surfaces 20, 20a, 20b of the implant components 16, 16a, 16b. The permanent connection magnets 36 of the first implant component 16a and the second implant component 16b are mounted on the facing surfaces 20, 20a, 20b and are aligned with respect to each other, such that a magnetic attractive force between the implant components 16, 16a, 16b holds the implant components 16, 16a, 16b in a fixed relative spatial relationship and compresses the folded tissue sections 18a, 180 for creating the surgical anastomosis and defining a through-going passage 22 from the oral lumen 140 to the aboral lumen 14a.

In the sectional view of Fig. 3B, internal fluid channels 38 are visible extending through the rigid sections 32 and connecting the rigid sections 32 to the joining sections 34. The fluid channels 38 allow fluid access to the convertible cavities of the joining sections 34 to inject a filling medium and to convert the implant components 16, 16a, 16b between a first deflated configuration and a second inflated configuration featuring different perimeters Pi, P2. The filling medium may comprise a viscous or hardening medium, such as a superabsorbent polymer solution to establish and maintain a shape of the implant components 16, 16a, 16b. However, the implant components 16, 16a, 16b may additionally or alternatively also comprise absorbing or hardening additives, such as a polymer powder, and the fluid channels 38 may also guide a filling liquid to the joining sections to interact with the absorbing or hardening additives.

Fluid ports (not shown) may be provided on the implant components 16, 16a, 16b to connect the implant components 16, 16a, 16b to fluid conduits in order to fill the convertible cavities in the joining sections 34 with the filling medium. The fluid channels 38 may form a closed loop along the perimeter Pi, P2 of the implant component 16, 16a, 16b, such that the filling medium may be distributed though the implant component 16, 16a, 16b. The fluid ports may enable a selective connection and disconnection of the fluid conduits and may be associated with valves to inhibit an outflow of the filling medium from the fluid channels 38 when the fluid conduit is disconnected. However, the external fluid conduits may also be disconnected after the viscosity of the filling medium has increased, e.g. via a hardening process, such that disconnection of the fluid conduits from the fluid ports is associated with limited outflow of the filling medium. By filling the convertible cavities with the filling medium, an outer diameter of the implant component 16, 16a, 16b may be increased by at least 10%, preferably by at least 20%, such as 30%, e.g. by expanding the outer diameter of the implant component 16, 16a, 16b by at least 10 mm, such as from about 30 mm to about 40 mm or from about 30 mm to about 60 mm.

In some embodiments, the implant component 16, 16a, 16b features multiple convertible cavities. For example, the implant component 16, 16a, 16b may feature a first fluid cavity to receive a filling medium to establish a perimeter P1, P2 of the implant component 16, 16a, 16b and may comprise a second fluid cavity to adjust a compressive force onto the folded tissue portions 18a, 180 or to adjust a shape of the implant component 16, 16a, 16b. The convertible fluid cavities, such as the second fluid cavity, may also be prepared with a hardening medium to expand or harden when exposed to natural fluids of the body, such that the shape or compressive force of the implant components 16, 16a, 16b onto the clamped tissue sections 18a, 180 is passively adjusted in a timeframe following the implantation by absorption of surrounding fluids, e.g. to passively adapt the implant 10 to tissue swelling.

In Figs. 3A, 3B the implant component 16, 16a, 16b is illustrated with extended rigid sections 32 interrupting the joining sections 34. However, the skilled person will appreciate that the rigid sections 32 may in principle also be formed by the connection elements, mounting elements, or fluid ports of the implant component 16, 16a, 16b and the joining sections may join the respective elements via a continuous convertible cavity forming a closed loop around the perimeter of the through-going passage. Further, the implant component 16, 16a, 16b may comprise elastic joining elements to bias the implant component 16, 16a, 16b towards an elliptic shape without affecting the convertibility of the implant component 16, 16a, 16b. Additionally or alternatively, mechanical adjusting mechanism may be provided to bias the implant components 16, 16a, 16b into a target shape or to maintain a perimeter of the through-going passage 22.

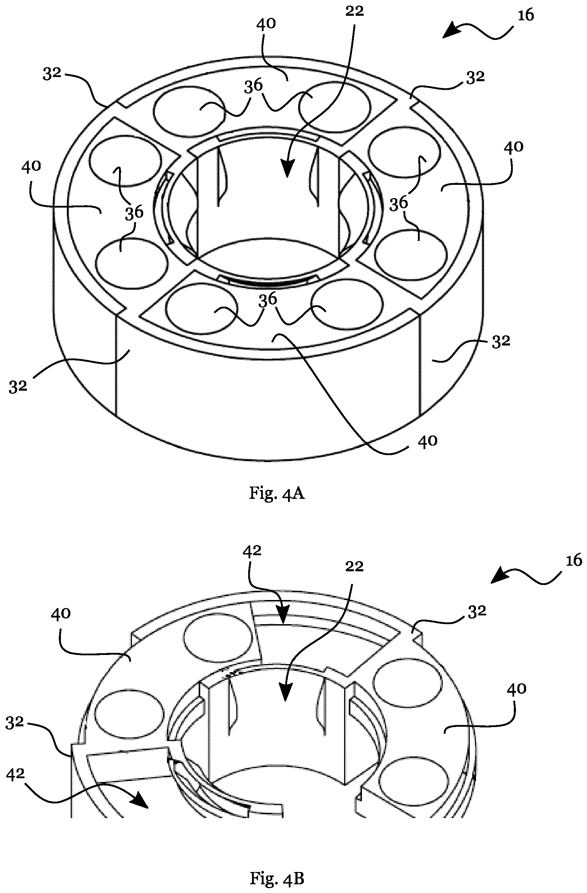

Figs. 4A and 4B schematically illustrate another example of a convertible implant component 16, 16a, 16b, wherein Fig. 4A illustrates the implant component 16, 16a, 16b in a first configuration featuring a first perimeter P1. Fig. 4B schematically illustrates the implant component 16, 16a, 16b in an expanded second configuration associated with a larger perimeter P2 with an isolated view of two rigid sections 32 of the implant component 16, 16a, 16b joined to each other.

The convertible implant component 16, 16a, 16b illustrated in Fig. 4A comprises four interconnected rigid sections 32, the rigid sections 32 mounting permanent connection magnets 36 to provide matching connection elements for connecting the implant component 16, 16a, 16b to a corresponding mirrored replica. Each rigid section 32 comprises a protruding tongue 40 and an accommodating groove 42, wherein the tongue 40 of each rigid section 32 protrudes into the accommodating groove 42 of an adjacent rigid section 32 and movably interconnects the rigid sections 32 as an adjustable joining section 34. The tongue 40 is in a form-locking engagement with the groove 42 such that a relative motion of the rigid sections 32 is limited to a distancing of adjacent rigid sections 32 along the perimeter Pi, P2 of the implant component 16, 16a, 16b, wherein the tongue 40 may be retracted from the groove 42 to adjust the perimeter P1, P2 of the implant component 16, 16a, 16b and convert the implant component 16, 16a, 16b as illustrated in Fig. 4B.

When the implant component 16, 16a, 16b is to be implanted, the implant component 16, 16a, 16b is expanded from the first storage/transport configuration depicted in Fig. 4A towards an expanded second configuration featuring a larger diameter P2 (partially illustrated in Fig. 4B). The rigid sections 32 of the implant component 16, 16a, 16b may be at least partially flexible, such that the tongue 40 may not jam within the groove 42 during the expansion of the implant component 16, 16a, 16b.

The implant component 16, 16a, 16b may be locked into the second configuration by any suitable mechanism, such as a filling of convertible cavities in the implant component 16, 16a, 16b or a mechanical locking mechanism, while the expansion of the implant component 16, 16a, 16b may be guided by the mechanical interlocking of the rigid sections 32.

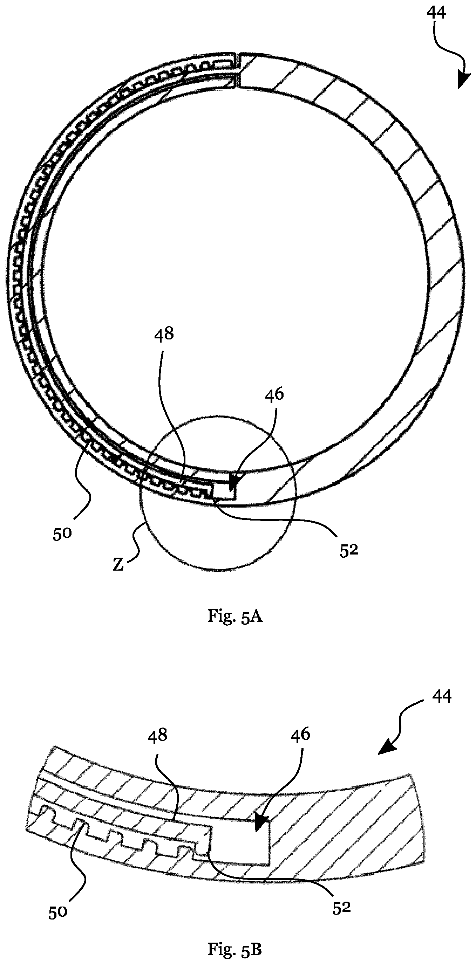

Figs. 5A and 5B illustrate a mechanical locking mechanism 44 for a convertible implant component 16, 16a, 16b with a general view in Fig. 5A and a zoomed detail view of the encircled area "Z" in Fig. 5B. The mechanical locking mechanism 44 is configured to lock a configuration of the convertible implant component 16, 16a, 16b when the perimeter Pi, P2 of the implant component 16, 16a, 16b is adjusted and to maintain an expanded perimeter P2 of the implant 10 for surgical anastomosis.

As illustrated in Fig. 5A, the mechanical locking mechanism 44 may comprise a ring-shaped body with a first end featuring a receiving cavity 46 and a second end with a protruding member 48. The protruding member 48 protrudes into the receiving cavity 46, such that the ring-shaped body forms a closed loop. The receiving cavity comprises protruding teeth 50 distributed around the perimeter Pi, P2 of the ring-shaped body and protruding into the cavity along a radial direction. The protruding member 48 features a pin 52 to protrude from the protruding member 48 along the radial direction and to interlock with the protruding teeth 50 of the mechanical locking mechanism 44 inside of the receiving cavity 46 to lock the shape of the mechanical locking mechanism 44 and to maintain a perimeter Pi, P2 of the mechanical locking mechanism 44.

The mechanical locking mechanism 44 may comprise elastic elements or be formed of an at least partially elastic ring-shaped body, such that the pin 52 and the teeth 50 are biased into mutual engagement when the mechanical locking mechanism 44 is converted from the first configuration into an expanded second configuration and to maintain a perimeter Pi, P2 of the implant component 16, 16a, 16b.

As illustrated in Fig. 5B, the teeth 50 may be asymmetrically shaped with chamfered portions to promote relative movement of the pin 52 and the teeth 50 in an expansion direction and to prevent movement of the pin 52 and the teeth 50 in the opposite contraction direction by a form locking engagement of the pin 52 with a substantially vertical flank of the teeth 50. Hence, the locking mechanism 44 may be attached to the implant component 16, 16a, 16b and may maintain an expanded configuration of the implant component 16, 16a, 16b after the implant component 16, 16a, 16b has been expanded for surgical anastomosis.

The skilled person will appreciate that the locking mechanism 44 may also comprise a plurality of segments each comprising receiving cavities 46 and protruding members 48 to form a closed loop with the plurality of segments joined to each other via their respective receiving cavities 46 and protruding members 48. The mechanical locking mechanism 44 may also be integrated with the rigid sections 32 to prevent collapse of the joining sections 34, such as by integrating the teeth 50 and the pin 52 in the tongue 40 and the accommodating section 42 of the rigid sections 32 in the example of Figs. 4A, 4B. Further, the locking mechanism 44 may be arranged inside of the implant components 16, 16a, 16b, such as within channels 38 extending through convertible cavities of the implant components 16, 16a, 16b.

Hence, the implant component 16, 16a, 16b may be mechanically expanded at the site of the surgical intervention and may maintain the expanded shape through the mechanical locking mechanism 44 for creating a surgical anastomosis with a selectable perimeter.

Preferably, the implant components 16, 16a, 16b according to embodiments of the invention comprise at least three segments, preferably four segments, whose spacing is adjustable and distributed in a regular polygon, such that an expansion of the implant component 16, 16a, 16b may be performed radially symmetric, e.g. with a radially symmetric expander. For example, mounting elements 54 of the implant component 16, 16a, 16b for engagement with corresponding mounting supports an applicator 24 may be aligned in a regular polygon to facilitate expansion of the implant 10 via an arrangement of radially symmetric implant manipulators 28a, 28b, wherein the radially symmetric arrangement may facilitate folding of the tissue sections 12a, 120 of the surrounding tissue over the implant components 16, 16a, 16b. However, the implant components 16, 16a, 16b may feature any suitable shape to define a through-going passage 22, such as an elliptical or polygonal shape.

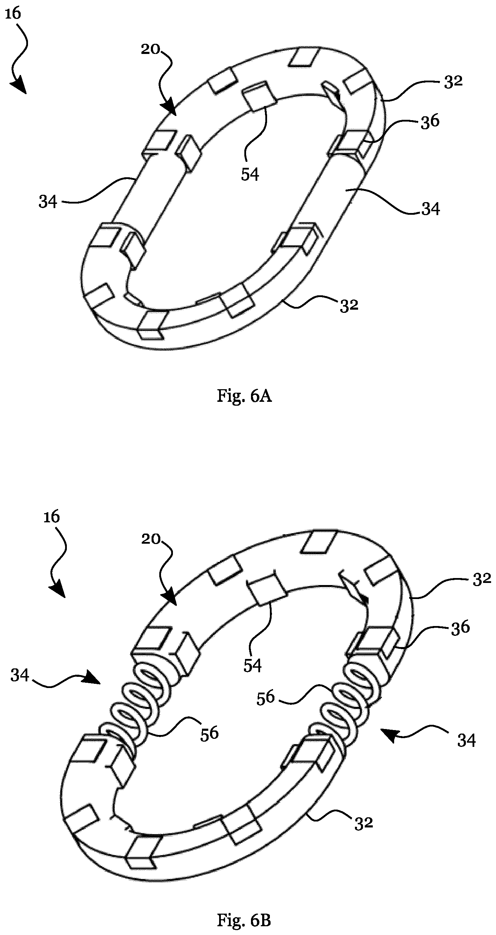

Fig. 6A schematically illustrates another example of a convertible implant component 16, 16a, 16b. The implant component 16, 16a, 16b comprises two semi-elliptical rigid sections 32 connected by flexible joining sections 34. The joining sections 34 may comprise convertible fluid channels 38 for receiving a filling medium for expanding the implant 10 along a radial direction by selectively spacing the rigid sections 32 apart from each other and establishing an elliptical through-going passage 22 with the rigid sections 32 and the convertible joining sections 34.

Each of the rigid sections 32 comprises a plurality of permanent connection magnets 36 as connection elements arranged on a facing surface 20 of the convertible implant component 16, 16a, 16b for connecting the implant component 16, 16a, 16b to a matching implant component 16, 16a, 16b and for creating a surgical anastomosis.

In addition, the implant component 16, 16a, 16b comprises a plurality of mounting elements 54 arranged on an inner perimeter Pi, P2 of the implant component 16, 16a, 16b and mounted to the rigid sections 32. The mounting elements 54 may be provided as permanent magnets to mount the implant to magnetic or magnetizable sections of an implant applicator 24 at the inner perimeter Pi, P2 of the convertible implant component 16, 16a, 16b.

As the implant component 16, 16a, 16b expands along one radial direction, it may be applied in a controlled manner using only two manipulator arms for holding the rigid sections 32 at a selectable distance and may thus simplify manipulation of the implant components 16, 16a, 16b for creating a surgical anastomosis.

Fig. 6B schematically illustrates a further example of a convertible implant component 16, 16a, 16b comprising two semi-elliptical rigid sections 32 connected by flexible joining sections 34. The flexible joining sections 34 comprise an elastic component 56 which biases the convertible implant component 16, 16a, 16b towards a target shape in an expanded, second configuration. Hence, the convertible implant component 16, 16a, 16b may be inserted through a lumen 14a in a contracted storage configuration and may expand at the site of the surgical anastomosis assisted via the spring force of the elastic component 56. The elastic component 56 may be housed in a flexible enclosure (not shown) to shield the surrounding tissue and the elastic component 56 from each other.

The elastic component 56 may be formed of a shape-memory alloy, such that a bias towards the target shape depends on a temperature of the alloy. Hence, the elastic component 56 may be compressed in a cold state and may expand when heated by the temperature of the surrounding lumen when the elastic component 56 crosses a transformation temperature of the shape-memory alloy.

The skilled person will appreciate that the biasing force of the elastic component 56 may be combined with any of the preceding examples of mechanisms for converting the convertible implant component 16, 16a, 16b between different configurations, such as convertible cavities for receiving a filling (hardening) medium or mechanical locking mechanisms 44. In some embodiments, the elastic component 56 may be provided for expanding the convertible implant component 16, 16a, 16b while additional mechanisms are provided for establishing a range of selectable perimeters Pi, P2 of the convertible implant component 16, 16a, 16b. For example, the elastic component 56 may be provided for expanding the convertible implant component 16, 16a, 16b and a mechanical locking mechanism 44 may prevent a contraction of the implant component 16, 16a, 16b. As another example, the elastic component 56 may be integrated with or form part of a convertible cavity and may bias the implant component 16, 16a, 16b towards an expanded configuration while a filling medium may be introduced into the convertible cavity to maintain or adjust a shape of the through-going passage 22 defined by the implant component 16, 16a, 16b in the expanded configuration.

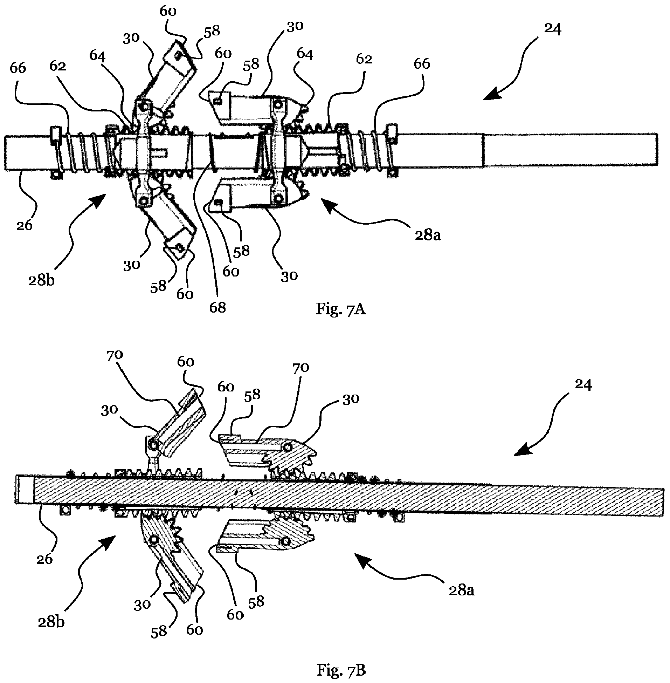

Fig. 7A and 7B schematically illustrate an example of an implant applicator 24 for a convertible implant 10 comprising two expandable implant components 16, 16a, 16b (not shown) with a side view and a cross-sectional view along the plane of projection of Fig. 7A, respectively. The implant applicator 24 comprises an elongated applicator body 26 as well as a first implant manipulator 28a and a second implant manipulator 28b arranged at different positions along the elongated applicator body 26 and facing each other.

The implant manipulators 28a, 28b each comprise two manipulator arms 30 arranged on opposite sides of the elongated manipulator body 26. The first implant manipulator 28a is configured to mount a first implant component 16a of the convertible implant 10 and the second implant manipulator 28b is configured to mount a second implant component 16b of the convertible implant 10 via mounting supports 58 on respective manipulator arms 30. The mounting supports 58 are arranged on radially outward faces of the manipulator arms 30 and close to facing tissue folding sections 60 of each manipulator arm 30 to mount matching implant components 16, 16a, 16b via corresponding mounting elements 54 on the inner perimeters Pi, P2 of the implant components 16, 16a, 16b.

The manipulator arms 30 are pivotally mounted on a implant manipulator base 62 via a worm gear mounting resulting from an engagement between a worm shaft toothing on the implant manipulator base 62 and a worm gear toothing 64 of the manipulator arms 30. Hence, by displacing the manipulator arms 30 with respect to the implant manipulator base 62 along the elongated applicator body 26 or by rotating the implant manipulator base 62, the manipulator arms 30 can be pivoted to radially displace the mounting supports 58 away from the elongated applicator body 26. As illustrated in Figs. 7A, 7B, the manipulator arms 30 may be pivoted individually, e.g. the manipulator arms 30 of the first implant manipulator 28a may be in a contracted state, while the manipulator arms 30 of the second implant manipulator 28a may be in an expanded state. The manipulator arms 30 may expand an implant component 16, 16a, 16b mounted on the mounting supports 58 radially towards an expanded configuration by the pivoting motion, to fold tissue sections 12a, 120 around facing surfaces 20a, 20b of the implant components 16, 16a, 16b by a radial strain on the surrounding tissue and to align the tissue sections 12a, 120 for creating the surgical anastomosis.

The implant manipulator base 62 of each implant manipulator 28a, 28b may be movable along the elongated applicator body 26 to approach the implant manipulators 28a, 28b to each other. Further, the implant manipulators 28a, 28b may be biased into a default position via coupling elements. For example as shown in Figs. 7A, 7B, the implant manipulator base 62 of each implant manipulator 28a, 28b may be coupled to the elongated applicator body 26 via elastic biasing elements 66, such that the implant manipulators 28a, 28b assume a default position in the absence of external forces. In addition, an elastic spacing element 68 may bias the implant manipulators 28a, 28b away from each other, such that the implant manipulators 28a, 28b are prevented from approaching each other, when the external forces do not exceed a force threshold defined by the elastic spacing element 68 and the biasing elements 66.

A drive mechanism (not shown) may be actuated to selectively approach the implant manipulators 28a, 28b towards each other for approaching the implant components 16, 16a, 16b and creating the surgical anastomosis. The drive mechanism may comprise a cable pull mechanism or hydraulic mechanism (not shown) to transfer a mechanical force from a proximal portion of the implant applicator 24 along the elongated applicator body 26 towards the implant manipulators 28a, 28b to adjust a position of the implant manipulators 28a, 28b. For example, a fluid conduit of the drive mechanism may connect to an expandable element coupling the implant manipulators 28a, 28b, e.g. as the elastic spacing element 68, such that by adjusting a filling level of the expandable element via the fluid conduits, the implant manipulators 28a, 28b may be selectively moved with respect to each other through hydraulic force transfer via the fluid conduit. Additionally or alternatively, a cable pull, e.g. a Bowden cable, may be provided to transmit a pulling force along the elongated applicator body 26 and to drive a motion of the implant manipulators 28a, 28b. Additional drive mechanisms, e.g. cable pull, pneumatic or hydraulic drive mechanisms, may be provided to adjust an angular position of the manipulator arms 30 and to expand the implant components 16, 16a, 16b mounted via the mounting supports 58.

As schematically illustrated in Fig. 7B, the facing tissue folding sections 60 of the manipulator arms 30 may comprise facing suction ports each coupled to fluid conduits 70 within the manipulator arms 30 to apply a suction force at the facing tissue folding sections 60. For example, a fluid conduit running along the elongated manipulator body 26 may be connected to each implant manipulator 28a, 28b to transmit a suction force from the proximal end of the implant applicator 24 to the implant manipulators 28a, 28b in order to hold folded tissue sections 18a, 180 (not shown) aligned over the implant components 16, 16a, 16b mounted on the mounting supports 58.

In addition, fluid conduits may run along the elongated applicator body 26 to fill convertible cavities of the implant components 16, 16a, 16b and to convert the implant components 16, 16a, 16b towards a deployment configuration featuring a target perimeter Pi, P2 for establishing a through-going passage 22 between the disconnected lumina 12a, 120 with customizable dimensions.

Hence, the implant applicator 24 may be employed to position and expand the implant components 16, 16a, 16b in the disconnected tissue sections 12a, 12, to fold the tissue sections 12a, 120 over the implant components 16, 16a, 16b, and to approach the implant components 16, 16a, 16b towards each other for clamping the folded tissue sections 18a, 180 and creating the surgical anastomosis.

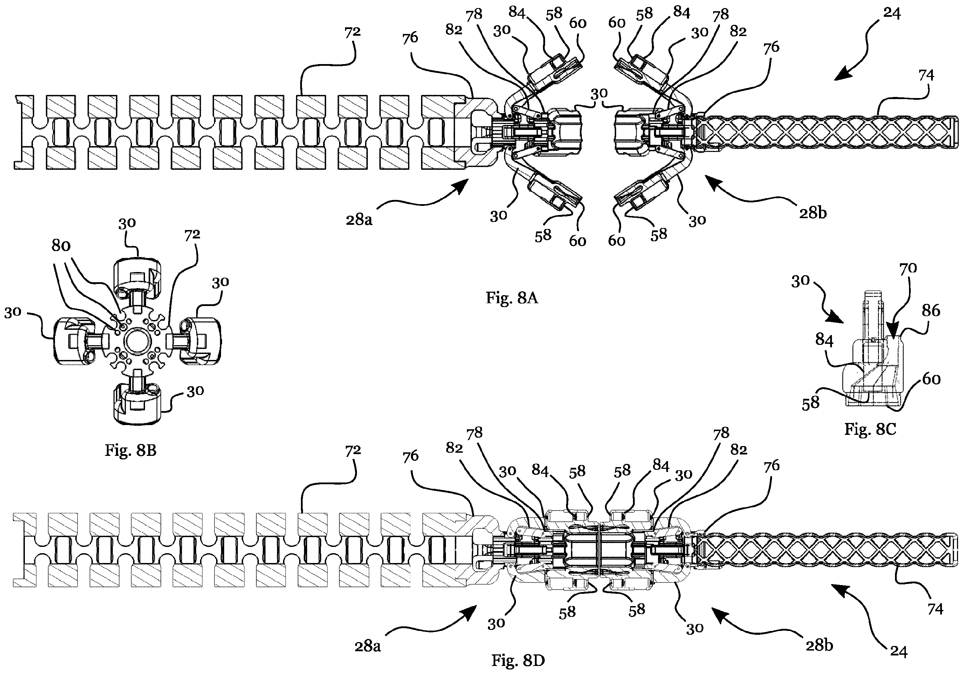

Fig. 8A-8D schematically illustrate another example of an implant applicator 24 for a convertible implant 10 comprising two expandable implant components 16, 16a, 16b (not shown). Fig. 8A illustrates a sectional view through the implant applicator 24, Fig. 8B shows a view of the implant applicator 24 from a proximal side of the implant applicator 24, Fig. 8C shows a semitransparent view of a manipulator arm 30, and Fig. 8D depicts a sectional view of the implant applicator of Fig. 8A in a closed state.

The implant applicator 24 comprises two implant manipulators 28a, 28b, a flexible overtube support body 72, and a distal spacing element 74 which mutually define an elongated flexible body along an axial direction to navigate through an anatomical lumen 14a, 140. In the example of Figs. 8A, 8B, 8D, the implant applicator 24 is configured as an overtube element to cover an endoscope (not shown), and the two implant manipulators 28a, 28b, the overtube support body 72, and the distal spacing element 74 each define a central through-going passage to mutually receive the endoscope. The overtube support body 72 connects a proximal side of the implant applicator 24 to the implant manipulators 28a, 28b, while the distal spacing element 74 defines a distal end of the implant applicator 24, such as to mount the implant applicator 24 to the distal end of the endoscope.

The overtube support body 72 may comprise a plurality of flexibly connected segments defining a plurality of channels 80 to guide fluid conduits or force transmission cables along the overtube support body 72 from a proximal portion of the implant applicator 24 towards the implant manipulators 28a, 28b and to transmit fluids and/or forces through an anatomical lumen 14a, 140, such as to control a state and/or position of the implant manipulators 28a, 28b and/or to manipulate the implant components 16, 16a, 16b. For example, a filling medium may be injected into the implant components 16, 16a, 16b through fluid conduits mounted on the channels of the overtube support body 72 to convert the implant components 16, 16a, 16b between different configurations. Additionally or alternatively, channels of the endoscope may be used to provide fluid connections between distal and proximal portions of the implant applicator 24.

The implant applicator 24 may further comprise a drive mechanism (not shown) to move one or both of the implant manipulators 28a, 28b towards each other, such that the mounting supports 58 for mounting the implant components 16, 16a, 16b may be approached towards each other for creating the surgical anastomosis. For example, a cable pull mechanism or hydraulic force transmission mechanism may transmit a force from a proximal portion of the implant applicator 24 along channels 80 of the overtube support body 72 to bias the implant manipulators 28a, 28b towards each other and to approach the implant components 16, 16a, 16b.

The implant manipulators 28a, 28b each comprise four manipulator arms 30 arranged in a radially symmetric configuration around the central through-going passage. The manipulator arms 30 are pivotally mounted on a pivot support 76 and coupled to a runner 78 with articulated arms 82. By adjusting the distance between the pivot support 76 and the runner 78, the manipulator arms 30 of each implant manipulator 28a, 28b may be jointly pivoted away from a central axis of the implant applicator 24, similar to the opening mechanism of an umbrella. The distance may be adjusted by pulling a cable pull to bias the pivot support 76 and the runner 78 towards each other, such that the articulated arms 82 induce a synchronous pivoting of the manipulator arms 30. The biasing force may be applied to the runner 78 and/or the pivot support 76 along channels 80 of the overtube support body 72. Pivoting the manipulator arms 30 may fold surrounding tissue sections over the facing tissue folding sections 60 to facilitate alignment of the tissue over the implant components 16, 16a, 16b and/or may expand the implant components 16, 16a, 16b mounted on the manipulator arms 30, such that the implant 10 may be manipulated for creating a surgical anastomosis in a simple manner.

The pivot points of the manipulator arms 30 should be arranged on a side of the manipulator arms 30 opposite to the facing tissue folding sections 60, such that when the manipulator arms 30 are pivoted away from the central body of the implant applicator 24, the implant components 16, 16a, 16b mounted on the mounting supports 58 can be continuously expanded while facing each other, such that a surgical anastomosis with a desired shape can be selectively created.

Mounting supports 58 can be arranged at the axial faces of the manipulator arms 30, such that the implant components 16, 16a, 16b are arranged to face each other on opposing faces of the manipulator arms 30, and may comprise mounting mechanisms 84 to selectively mount the implant components 16, 16a, 16b. For example, the mounting mechanisms 84 may comprise a permanent magnet or electromagnet to attract magnetic mounting elements 54 on the implant components 16, 16a, 16b. The implant components 16, 16a, 16b may then be selectively disconnected by reducing or inverting an attractive force between the mounting mechanism 84 and the mounting element 54, e.g. by mechanically retracting the permanent magnets or by adjusting a current of the electromagnet in the mounting mechanism 84. Hence, the implant components 16, 16a, 16b may be releasably mounted on the axial faces of the manipulator arms 30 to engage each other when the implant manipulators 28a, 28b approach each other.

Additionally, the facing tissue folding sections 60 of the implant manipulators 28a, 28b may comprise suction ports to apply a suction force to fold tissue sections 18a, 180 over the implant components 16, 16a, 16b. The manipulator arms 30 may comprise a fluid conduit connector 86 to receive a fluid conduit and to apply a suction force on the facing tissue folding section 60 via an internal fluid conduit 70 going through the manipulator arm 30. For example, a suction force may be applied through conduits mounted on the flexible overtube support body 72 and fluid conduits 70 in the manipulator arms 28a, 28b.

The folded tissue sections 18a, 180 may be held over the implant components 16, 16a, 16b mounted on the mounting supports 58 by the suction force applied at the facing tissue folding sections 60, such that the folded tissue sections 18a, 180 are aligned for engagement with the implant component 16, 16a, 16b along the relative displacement direction of the implant manipulators 28a, 28b. The implant components 16, 16a, 16b may then be approached towards each other to clamp the folded tissue sections 18a, 180 and engage matching connections elements of the implant components 16, 16a, 16b for creating the surgical anastomosis.

Preferably, the implant manipulators 28a, 28b define at least three, preferably four, mounting supports 58 which are radially displaceable away from a central axis of the implant manipulators 28a, 28b, such that surrounding tissue sections 12a, 120 and mounted implant component 16, 16a, 16b are uniformly expanded to form a regularly shaped through-going passage 22 for creating a substantially elliptical, in particular substantially circular, or regular polygon shaped surgical anastomosis.

However, the implant components 16, 16a, 16b may also be approached towards each other to clamp the folded tissue sections 18a, 180 before the implant components 16, 16a, 16b are expanded towards an expanded configuration, and the implant components 16, 16a, 16b may be expanded only after the matching connections elements of the implant components 16, 16a, 16b are engaged, and the implant components 16, 16a, 16b may be expanded without radially expanding the implant manipulators 28a, 28b.

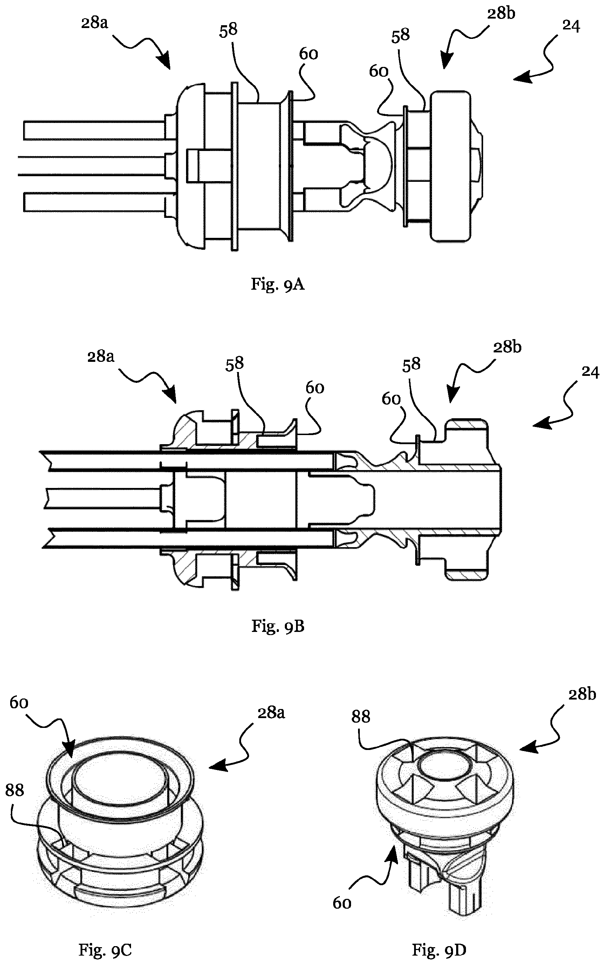

Figs. 9A-9D illustrate another example of an implant applicator 24 comprising a first implant manipulator 28a and a second implant manipulator 28b for mounting a convertible implant 10 comprising two expandable implant components 16, 16a, 16b (not shown). Figs. 9A and 9B illustrate a side view and a cross-sectional view of the implant manipulators 28a, 28b, respectively. Figs. 9C and 9D illustrate isolated perspective views of the first, proximal implant manipulator 28a and second, distal implant manipulator 28b, respectively.

Convertible implant components 16, 16a, 16b may be mounted on mounting supports 58 of the first implant manipulator 28a and the second implant manipulator 28b. The mounting supports 58 are configured as a circumferential bracket arranged at facing tissue folding sections 60, such that folded tissue sections 18a, 180 are held over the implant components 16, 16a, 16b when attached to the facing tissue folding sections 60. The implant components 16, 16a, 16b may be held in place by a mounting mechanism 84, which can be installed in manipulation channels 88 of the implant manipulators 28a, 28b, such as permanent magnets mounted within the manipulation channels 88 and operable to selectively adjust a magnetic attractive force between the implant components 16, 16a, 16b and the implant manipulators 28a, 28b.

Each of the implant manipulators 28a, 28b comprises an elliptical suction port at the facing tissue folding sections 60 of the implant manipulator 28a, 28b for applying a suction force along a closed loop and for aligning the folded tissue sections 18a, 180 continuously over the implant components 16, 16a, 16b.

The implant manipulators 28a, 28b are movable relative to each other, wherein the second implant manipulator 28b is configured to partially slide into a central through-going passage 22 of the first implant manipulator 28a to approach the implant manipulators 28a, 28b, for pulling the second implant manipulator 28b towards the first implant manipulator 28a and approaching the convertible implant components 16, 16a, 16b.

The implant manipulators 28a, 28b may further provide an implant expansion mechanism to selectively expand the implant components 16, 16a, 16b. For example, fluid conduits may connect to the implant components 16, 16a, 16b to selectively fill fluid cavities in the implant components 16, 16a, 16b with a filling medium and to expand the implant components 16, 16a, 16b. The third conduits may be guided through manipulation channels 88 of the implant manipulators 28a, 28b to avoid increasing an outer perimeter of the implant applicator 24. However, fluid conduits for manipulating the implant components 16, 16a, 16b may also run along the outer perimeter of the implant manipulators 28a, 28b.

A surgical anastomosis may be created using the applicator 24 by inserting the second implant manipulator 28b into an oral lumen 140 while the first implant manipulator 28a is located within an aboral lumen 14a and folding respective tissue sections of the surrounding tissue 120, 12a over the facing tissue folding sections 60 of the implant manipulators 28a, 28b. A suction force may be applied via the suction ports of the facing tissue folding sections 60 to hold the folded tissue sections 18a, 180 over the implant components 16, 16a, 16b. The implant components 16, 16a, 16b may then be approached by moving the implant manipulators 28a, 28b towards each other, such that the second implant manipulator 28b partially slides into the through going passage of the first implant manipulator 28 a to clamp the folded tissue sections 18a, 180 between the implant manipulators 28a, 28b.

By selectively disconnecting the implant components 16, 16a, 16b from the implant manipulators 28a, 28b, and connecting the implant components 16, 16a, 16b via matching connection elements, the surgical anastomosis may be created in a first configuration associated with a first perimeter P1 of a through-going passage 22. For example, by disconnecting a mounting mechanism of the implant manipulators 28a, 28b permanent connection magnets 36 of the implant components 16, 16a, 16b may connect the implant components 16, 16a, 16b to each other by an attractive magnetic force to apply the implant 10 and form the surgical anastomosis.

An implant expanding mechanism may then be actuated to synchronously expand the implant components 16, 16a, 16b from the first configuration towards a second configuration featuring a larger perimeter P2, e.g. by filling both implant components 16, 16a, 16b with a filling medium. Hence, the convertible implant 10 may be applied for creating a surgical anastomosis without a radially expanding component of the implant applicator 24, such that the number of movable implant applicator components can be reduced.

The description of the preferred embodiments and the figures merely serve to illustrate the invention and the beneficial effects associated therewith, but should not be understood to imply any limitation. The scope of the invention is to be determined solely by the appended claims.

### LIST OF REFERENCE SIGNS

- 10: implant
- 12a, 120: disconnected aboral and oral bowel sections
- 14a, 140: aboral and oral lumen
- 16: implant component
- 16a, 16b: first and second implant component
- 18a, 180: folded tissue sections
- 20, 20a, 20b: axial face/facing surface of the first and second implant component
- 22: through-going passage
- 24: implant applicator
- 26: elongated applicator body
- 28a, 28b: first and second implant manipulator
- 30: manipulator arm
- 32: rigid section
- 34: joining section
- 36: permanent connection magnets
- 38: fluid channels
- 40: tongue
- 42: accommodating groove
- 44: mechanical locking mechanism
- 46: receiving cavity
- 48: protruding member
- 50: teeth
- 52: pin
- 54: mounting elements
- 56: elastic element
- 58: mounting support
- 60: facing tissue folding sections
- 62: implant manipulator base
- 64: worm gear toothing
- 66: elastic biasing elements
- 68: elastic spacing element
- 70: fluid conduit
- 72: fexible overtube support body
- 74: distal spacing element
- 76: pivot support
- 78: runner
- 80: channels
- 82: articulated arm
- 84: mounting mechanism
- 86: fluid conduit connector
- 88: manipulation channel

## Claims

1. An implant (10) for surgical anastomosis, the implant (10) comprising a first implant component (16, 16a) and a second implant component (16, 16b),
wherein the first implant component (16, 16a) and the second implant component (16, 16b) comprise matching connection elements (36) configured to hold the first implant component (16, 16a) and the second implant component (16, 16b) in a fixed relative spatial relationship and/or to compress body tissue (12a, 120, 18a, 180) arranged between the first implant component (16, 16a) and the second implant component (16, 16b),
wherein the first implant component (16, 16a) and the second implant component (16, 16b) form a through-going passage (22) when the implant (10) is deployed for surgical anastomosis, and
wherein the first implant component (16, 16a) and the second implant component (16, 16b) are convertible between a first configuration and a second configuration, the first configuration and the second configuration defining different perimeters (Pi, P2), to establish a perimeter (Pi, P2) of the through-going passage (22).

2. The implant (10) of claim 1, wherein the first implant component (16, 16a) and the second implant component (16, 16b) are configured to maintain a perimeter (Pi, P2) selected from a range of supported perimeters (Pi, P2) when the implant (10) is deployed for surgical anastomosis.

3. The implant (10) of any one the preceding claims, wherein the first implant component (16, 16a) and the second implant component (16, 16b) are configured to transfer and/or to distribute compressive forces on body tissue (18a, 180) held between the first implant component (16, 16a) and the second implant component (16, 16b), such that a healing process of the body tissue (18a, 180) is stimulated along a closed loop around the perimeter (Pi, P2) of the through-going passage (22) when the implant (10) is deployed for supporting a surgical anastomosis.

4. The implant (10) of any one the preceding claims, wherein the first implant component (16, 16a) and the second implant component (16, 16b) comprise an implant body circumscribing the through-going passage (22) in a closed loop, wherein the implant body defines the inner perimeter (Pi, P2) in the first configuration and in the second configuration; wherein the matching connection elements (36) are in particular fixedly attached to the implant body.

5. The implant (10) of any one of the preceding claims, wherein the first implant component (16, 16a) and the second implant component (16, 16b) comprise a plurality of rigid sections (32) interconnected by adjustable joining sections (34) , wherein the first implant component (16, 16a) and the second implant component (16, 16b) are convertible between the first configuration and the second configuration by adjusting the joining sections (34) to establish a different spacing between the plurality of rigid sections (32) in the first configuration and in the second configuration,
wherein the joining sections (34) are in particular configured to maintain the spacing between the plurality of rigid sections (32) when the perimeter (Pi, P2) of the through-going passage (22) is established, and/or
wherein the joining sections (34) in particular comprise a shape setting element (38, 44, 56), in particular a convertible cavity for receiving a filling medium and/or a locking mechanism (44) and/or a compressible elastic element (56), to establish the perimeter (Pi, P2) of the through-going passage (22).

6. The implant (10) of any one the preceding claims, wherein the matching connection elements (36) of at least one of the first implant component (16, 16a) and the second implant component (16, 16b) comprise a permanent magnet (36) to magnetically attract a magnetizable portion, in particular an inversely polarized permanent magnet (36), of the respective other matching connection element (36).

7. The implant (10) of any one the preceding claims, the first implant component (16, 16a) and the second implant component (16, 16b) further comprising mounting elements (54) to mount the first implant component (16, 16a) and the second implant component (16, 16b) to an implant applicator (24) on the inner perimeter (P1, P2) of the first implant component (16, 16a) and the second implant component (16, 16b), respectively.

8. A kit comprising the implant (10) of any one of the preceding claims and an implant applicator (24) comprising relatively displaceable mounting supports (58) to releasably mount the first implant component (16, 16a) and the second implant component (16, 16b) in a spatially separated arrangement, and to approach the first implant component (16, 16a) and the second implant component (16, 16b) to each other for surgical anastomosis.

9. An implant applicator (24) for clamping surrounding tissue (12a, 120, 18a, 180) sections with a pair of implant components (16, 16a, 16b) to create a surgical anastomosis, the implant applicator (24) comprising:
an elongated applicator body (26),
a pair of implant manipulators (28a, 28b) configured to be displaced with respect to each other along the applicator body (26), and
a drive mechanism to move one or both of the implant manipulators (28a, 28b) of the pair of implant manipulators (28a, 28b) towards each other,
wherein each implant manipulator (28a, 28b) (28a, 28b) of the pair of implant manipulators (28a, 28b) comprises a mounting support (58) to selectively hold a respective implant component (16, 16a, 16b) of the pair of implant components (16, 16a, 16b), and
wherein the implant applicator (24) further comprises an implant expanding mechanism for selectively increasing an inner perimeter (Pi, P2) of one or both implant components (16, 16a, 16b) of the pair of implant components (16, 16a, 16b) mounted on the mounting support (58).

10. The implant applicator (24) of claim 9, wherein each implant manipulator (28a, 28b) of the pair of implant manipulators (28a, 28b) further comprises a tissue folding section (60) for at least partially folding tissue (12a, 120, 18a, 180) of a lumen (14a, 140) surrounding the implant manipulator (28a, 28b), such that the tissue (12a, 120, 18a, 180) of the lumen (14a, 140) is aligned for engagement with the implant component (16, 16a, 16b) held by the implant manipulator (28a, 28b) along the displacement axis of the elongated applicator body (26);
wherein each implant manipulator (28a, 28b) of the pair of implant manipulators (28a, 28b) in particular comprises a suction port at the tissue folding section (60), and wherein the implant applicator (24) comprises a fluid conduit (70) for applying a suction force to the suction port.

11. The implant applicator (24) of claim 9 or 10, wherein the implant expanding mechanism is configured to radially displace the mounting support (58) of one or both of the pair of implant manipulators (28a, 28b) away from the elongated applicator body (26), wherein the pair of implant manipulators (28a, 28b) in particular comprises pivotally hinged manipulator arms (30) to pivot with respect to the elongated applicator body (26) such that the mounting support (58) is radially displaced away from the elongated applicator body (26); and/or
wherein the implant expanding mechanism comprises
- fluid conduits to transfer a fluid to a cavity (38) of each implant component (16, 16a, 16b) of the pair of implant components (16, 16a, 16b), and/or
- an actuation assembly coupled to the pair of implant manipulators (28a, 28b) and configured to change a radial distance of the respective mounting support (58) to the elongated applicator body (26) by mechanical displacement of the pair of implant manipulators (28a, 28b).

12. The implant applicator (24) of any one of claims 9 to 11, further comprising a release actuator (84) to release a mounting of the implant components (16, 16a, 16b) to the mounting supports (58) of the implant manipulators (28a, 28b); and/or
wherein the mounting supports (58) comprise magnetic or magnetizable portions to mount the respective implant components (16, 16a, 16b) via attracting magnetic forces, in particular a permanent magnet or an electromagnet;
wherein the release actuator (84) is in particular configured to mechanically retract or pivot the magnetic or magnetizable portions to reduce a magnetic force exerted by the mounting supports (58) onto the implant components (16, 16a, 16b) and/or to electrically invert or reduce a magnetic force exerted by the mounting supports (58) onto the implant components (16, 16a, 16b).

13. The implant applicator (24) of any one of claims 9 to 12, wherein the drive mechanism comprises
a fluid conduit for applying a hydraulic or pneumatic force from the proximal portion of the implant applicator (24) to a distal portion of the implant applicator (24); and/or
a cable pull to transmit a mechanical force from the proximal portion of the implant applicator (24) to a distal portion of the implant applicator (24); and/or
an elastic element (66, 68) mounted on the elongated applicator body (26) and biasing at least one implant manipulator (28a, 28b) of the pair of implant manipulators (28a, 28b).

14. The implant applicator (24) of any one of claims 9 to 13, wherein the elongated applicator body (26) comprises an accommodating passage to mount the implant applicator (24) on the outer face of an endoscope, and/or
wherein the implant applicator (24) comprises an imaging device configured to transmit an image from the distal portion of the implant applicator (24) to a proximal portion of the implant applicator (24); and/or
wherein the elongated applicator body (26) comprises flexible or flexibly connected segments to navigate through an anatomical lumen (14a, 140); and/or
wherein the outer diameter of the implant applicator (24) is smaller than 60 mm, in particular smaller than 40 mm, preferably smaller than 30 mm, in a navigation configuration of the implant applicator (24) to navigate through an anatomical lumen (14a, 140).

15. A kit of an implant (10) according to any one of claims 1 to 7 and an implant applicator (24) according to any one of claims 9 to 14, wherein the implant applicator (24) is configured for surgical anastomosis using the implant (10).
